# EUROPEAN PATENT SPECIFICATION

(11) **EP 3 125 742 B1**
(45) Date of publication and mention of the grant of the patent: **29.08.2018**
(21) Application number: 15802335.8
(22) Date of filing: 13.05.2015
(51) Int. Cl.: G01B 9/02, A61B 3/10, A61B 1/07, A61B 3/12, A61B 5/00

(54) **BACK REFLECTION MINIMIZATION FOR OCT PROBES**
RÜCKREFLEXIONSMINIMIERUNG FÜR OCT-SONDEN
RÉDUCTION AU MINIMUM D'ÉCHO DE FOND POUR SONDES OCT

(30) Priority: 06.06.2014 US 201414298233
(43) Date of publication of application: 08.02.2017
(73) Proprietor: Novartis AG, 4056 Basel (CH)
(72) Inventor: WHEATLEY, Barry Lynn, Oceanside, California 92057 (US); PARTO, Kambiz, Laguna Hills, California 92653 (US); SCHMIDTLIN, Edouard, San Francisco, California 94114 (US)
(74) Representative: Hanna Moore + Curley
(86) International application number: PCT/US2015/030630
(87) International publication number: WO 2015/187325

(56) References cited:
- US-A1- 2003 004 412
- US-A1- 2009 208 143
- US-A1- 2012 226 267
- US-A1- 2012 226 267
- US-B1- 6 340 248
- US-B1- 6 485 413

## Description

### TECHNICAL FIELD

The present disclosure relates to apparatuses and methods for minimizing back reflection in an OCT probe.

### BACKGROUND

Optical Coherence Tomography (OCT) systems are used to capture and generate three-dimensional images of patient tissue layers. These systems include OCT probes that often invasively penetrate tissue to obtain visualization of tissue within a patient. In ophthalmology, OCT probes are used to obtain detailed images of tissue about the eye or even forming a part of the eye, such as the retina.

In use, an optical light beam is directed through the probe at the tissue. A small portion of this light reflects from sub-surface features of the tissue and is collected with a collector through the same probe. Most light is not reflected but, rather, diffusely scatters at large angles. In conventional imaging, this diffusely scattered light contributes background that obscures an image. However, in OCT, a technique called interferometry records the optical path length of received photons, and provides data that rejects most photons that scatter multiple times before detection. This results in images that are clearer and that extend in the depth of the tissue.

In some instances, some of the light is reflected from a first (input) surface of the lens and back along a return path to a collector, thereby over-saturating a light signal reflected from tissue and traveling back along the return path through the lens toward the collector. A conventional solution to this problem is to have the first surface of the lens fabricated with an angle from the normal face of the lens. This causes the lens' first surface to reflect light away from the return path, thereby reducing a chance of undesirable back reflection being received at the collector.

However, because of its small size, fabricating the lens with this angled surface can be challenging and relatively expensive. In addition to the small dimensional scale, the length of the lens must be held to tight tolerances, as it is critical to the optical performance. This compounds the manufacturing challenges.

The present disclosure addresses one or more deficiencies in the prior art.

Reference is made to the cited document US2012/226267 relating to a surgical instrument system for ophthalmic surgery in an eye is provided, comprising: an OCT apparatus including an interferometer; an optical fiber coupled to the OCT apparatus and extending a probe arm of the interferometer; a hand tool comprising: a hand piece, a tube extending away from the hand piece and comprising a distal end portion having a longitudinal axis, wherein a distal portion of the optical fiber is received within the tube; a beam emitter coupled to a tip end of the optical fiber and configured to emit an OCT measuring beam into an emission direction; and an actuator configured to change the emission direction of the OCT measuring beam relative to a tip end of the distal end portion.

### SUMMARY

It will be appreciated that the scope of the invention is in accordance with the claims. According there is provided an OCT probe in accordance with claim 1. Optional features are provided in accordance with the dependent claims..

In an aspect, the distal side of the lens includes a perimeter edge in a first plane and the proximal side includes a perimeter edge in a second plane, the first and second planes being substantially parallel to each other. In an aspect, the lens comprises an outer periphery, the proximal side and the distal side being arranged so that the first and second planes form substantially a right angle with the outer periphery. In an aspect, at least one of the proximal side and the distal side is planar. In an aspect, the distal segment is formed of a bend in the cannula. In an aspect, the cannula is sized to penetrate the globe of an eye to image tissue in the eye. In an aspect, the OCT probe includes an actuation system configured to displace the fiber in the lumen of the main body segment in a direction transverse to a plane through the first central axis and the second central axis.

In another exemplary aspect, the present disclosure is directed to an OCT probe for imaging patient tissue. The probe includes a cannula extending from the probe housing and arranged to penetrate patient tissue, with the cannula having an elbow formed therein dividing the cannula into a main body segment and a distal segment. The main body segment may have a lumen defining a first central axis. The distal segment may have a lumen defining a second central axis that is angled from the first central axis. A selectively displaceable light-carrying fiber is disposed within the main body segment of the cannula. The fiber has a distal end and is arranged to emit light from the distal end. The probe also includes a lens disposed in the distal segment of the cannula. The lens includes a proximal side and a distal side, with the proximal side being disposed relative to the fiber so that light emitted through the fiber and reflected from the proximal side reflects at an angle misaligned with the fiber.

In an aspect, the distal side of the lens includes a perimeter edge in a first plane and the proximal side of the lens includes a perimeter edge in a second plane, the first and second planes being substantially parallel to each other. In an aspect, the lens comprises an outer periphery, the proximal side and the distal side being arranged so that the first and second planes form substantially a right angle with the outer periphery. In an aspect, at least one of the proximal side and the distal side is planar. In an aspect, the cannula is sized to penetrate the globe of an eye to image tissue in the eye. In an aspect, the lens has a width less than about 2mm and a length less than about 2mm. In an aspect, the elbow is a bend in the cannula. In an aspect, the present disclosure includes an actuation system configured to displace the fiber in the lumen of the main body segment in a direction transverse to a plan through the first central axis and the second central axis.

In yet another exemplary aspect, the present disclosure is directed to a method of manufacturing an OCT probe for imaging patient tissue. The method may include bending a cannula to form a main body segment and a distal segment, the main body segment having a lumen defining a first central axis, the distal segment having a lumen defining a second central axis that is angled from the first central axis, the cannula being sized and arranged to penetrate patient tissue; inserting the cannula into a probe housing forming a handle configured to be grasped and manipulated by a user; and inserting a lens into the distal segment, the lens having a proximal side and a distal side, the distal side having a peripheral edge in a first plane and the proximal side having a peripheral edge in a second plane, the first and second planes being substantially parallel to each other. In an aspect, the method includes introducing an optical fiber into the main body segment of the cannula for emitting light through the lens.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTION OF THE DRAWINGS

The accompanying drawings illustrate embodiments of the devices and methods disclosed herein and together with the description, serve to explain the principles of the present disclosure.
Fig. 1 is a block diagram of an exemplary OCT imaging system in accordance with an aspect of the present disclosure.
Fig. 2 is a stylized illustration of a cross-sectional view of an OCT probe in accordance with an aspect of the present disclosure.
Fig. 3 is a detailed stylized illustration of a distal portion of the OCT probe of Fig. 2 in accordance with an aspect of the present disclosure.
Fig. 4 is an illustration of a lens usable in the OCT probe of Fig. 2 in accordance with an aspect of the present disclosure.
Fig. 5 is an illustration of a lens usable in the OCT probe of Fig. 2 in accordance with an aspect of the present disclosure.
Fig. 6 is an illustration of a lens usable in the OCT probe of Fig. 2 in accordance with an aspect of the present disclosure.
Fig. 7 is a stylized illustration of a cross-sectional view of an OCT probe in accordance with an aspect of the present disclosure.
Fig. 8 is a flow chart showing an exemplary method of manufacturing an OCT probe in accordance with an aspect of the present disclosure.

### DETAILED DESCRIPTION

For the purposes of promoting an understanding of the principles of the present disclosure, reference will now be made to the exemplary embodiments illustrated in the drawings, and specific language will be used to describe the same. It will nevertheless be understood that no limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. For simplicity, in some instances the same reference numbers are used throughout the drawings to refer to the same or like parts.

The present disclosure relates generally to OCT probes, OCT systems, and associated methods. The probe includes a lighting system including a lens and an optical fiber that directs light through the lens and provides a return path for reflected light that passes back through the lens. The OCT probes, OCT systems, and methods may reduce or minimize the back-reflected light through the use of an angled cannula that situates a lens at an angle, thereby improving the optical signal to noise ratio. That is, as described below, the cannula is angled so that a distal segment is misaligned from a main body segment. The lens itself is disposed in the distal segment so that its optical axis is angled relative to a main body of the cannula. The relative angle between the axis of the cannula and the optical axis of the lens causes light that reflects from the first surface of the lens to be reflected back at an angle such that it does not travel in the same path as tissue-reflected light.

In addition, the cannula disclosed herein may alleviate the problem of fabricating a small angle on the face of the lens used for OCT probes. Accordingly, both sides of the lens could be formed to be substantially parallel, without relative angles. This may achieve the same optical affect as having the first surface of the lens with an angle as described earlier. The advantage is that fabricating a short segment of a cannula with an angle may be easy and inexpensive to fabricate.

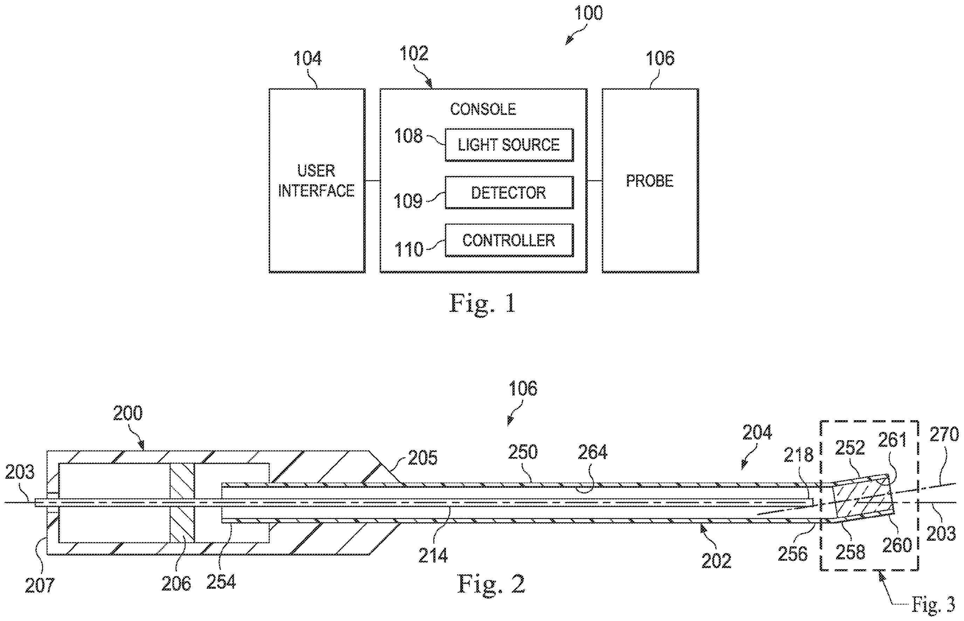

Fig. 1 shows a block diagram of an exemplary OCT imaging system 100. The system 100 includes a console 102, a user interface 104, and an OCT probe 106. The console 102 includes an OCT engine including, among other elements, a light source 108, a detector 109, and a controller 110. The light source 108 is configured to provide near-infrared light that can be reflected from the target biological tissue through the OCT probe 106. In some embodiments, the light source 108 is made up of super-luminescent diodes, ultra-short pulsed lasers, or super-continuum lasers that provide relative long wavelength light. The detector 109 is configured to receive reflected light from the OCT probe 106 and convert the reflected light into signals representing the images represented by the reflected light. The controller 110 may include a processor and memory that may include an executable program for operating the light source 108, the user interface 104, and the OCT probe 106, for processing light detected at the detector 109, and for executing and performing functions and processes to carry out an OCT imaging procedure.

In some embodiments, the user interface 104 is carried on or forms a part of the console 102. The user interface 104 may be a display configured to present images to a user or a patient, such as images of tissue scanned by the probe 106 during an OCT imaging procedure. The user interface 104 also may include input devices or systems, including by way of non-limiting example, a keyboard, a mouse, a joystick, dials, and buttons, among other input devices.

The OCT probe 106 is sized and shaped to be handled by a surgeon and to protrude into a body of the patient. In the embodiment shown, it is in electrical and optical communication with the console 102 and configured to present light from the light source 108 onto patient tissue for the purpose of imaging the tissue. Some OCT probe embodiments are configured to invasively penetrate a globe of an eye to capture images of eye tissue, such as retinal tissue.

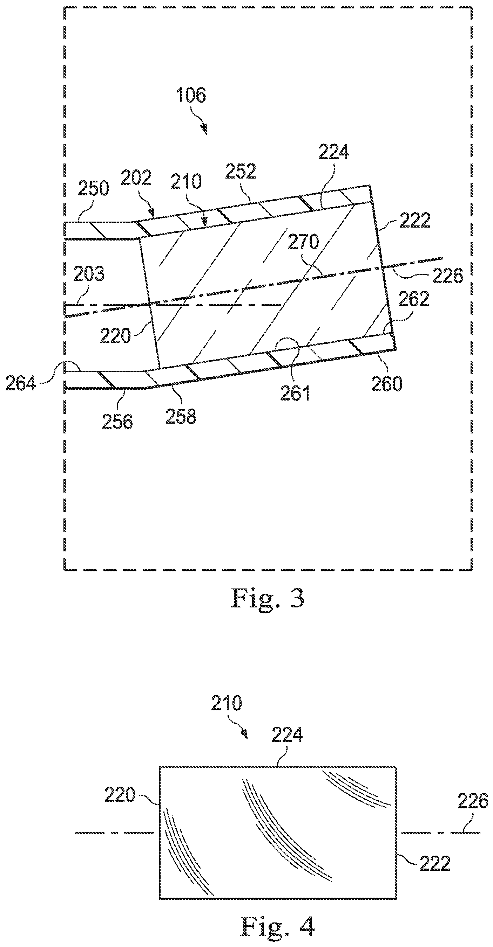

Fig. 2 shows a stylized cross-sectional illustration of an exemplary OCT probe 106, and Fig. 3 shows a distal end region of the OCT probe 106 in greater detail. As will be described in greater detail below, the OCT probe 106 includes a distal segment that houses a lens that may be relatively easy to manufacture and that may reduce or minimize reflection of light from the lens back toward the collector 109.

The OCT probe 106 includes a probe housing 200, a cannula 202, a lighting system 204, and an actuation system 206. The probe housing 200 is configured to be grasped and manipulated by a surgeon during an OCT procedure. It may be shaped as a handle or grip and may house other elements of the OCT probe 106. It includes a distal end 205 and a proximal end 207. The probe 106 may connect to the console (102 in Fig, 1) via a light carrier, such as a fiber or other connector extending from the probe proximal end 207.

The cannula 202 projects from the distal end 205 of the probe housing 200 and is configured and arranged to penetrate patient tissue in order to obtain an OCT image. With reference to both Figs. 2 and 3, the cannula 202 includes an elbow dividing it into a main body segment 250 and a distal segment 252. The main body segment 250 includes a proximal end 254 disposed within and supported by the probe housing 200 and includes a distal portion 256.

The main body segment 250 forms and is defined by the length of a lumen 264 and, in some embodiments, extends nearly the complete length of the cannula 202, such as more than 90% of the total length of the cannula. In other embodiments, the main body segment extends more than 80% of the cannula 202. Other lengths are contemplated. The lumen 264 includes a central axis 203 formed by the inner surface of the lumen 264. The lumen 264 houses or carries an optical fiber 214 that may carry light both toward the distal end for emission from the probe 106 and carry tissue-reflected light toward the proximal end for image processing. The optical fiber is discussed further below.

As shown in Figs. 2 and 3, the distal segment 252 includes a proximal portion 258, a distal end 260, and a lumen 261. The proximal portion 258 is disposed adjacent to and extends from the distal portion 256 of the main body segment 250. The distal end 260 forms an opening 262 to the lumen 261 through which light may pass during an OCT scan. The distal segment 252 may be connected to the main body segment 250 in any manner, and in some embodiments, is formed by simply bending the distal end of the cannula 202 to form an angled portion.

The distal segment 252 includes a distal segment central axis 270 that is defined by the lumen 261. As can be seen, the lumen 261 of the distal segment 252 is formed relative to the lumen 264 of the main body segment 250 so that the central axis 203 of the main body segment 250 is angled relative to the central axis 270 of the distal segment 252. The angle of the central axis 203 relative to the axis 270 may vary depending on the utility of the OCT probe. In some embodiments, the angle is within a range of about 4 and 20 degrees. In some embodiments, the angle is within a range of about 7 and 9 degrees, and in some embodiments the angle is about 8 degrees. As such, the central axis 203 and the central axis 270 are not coaxial. The length of the distal segment 252 may vary and, in some embodiments, has a length measured along the central axis between about 0.5 and 3mm. In some embodiments, the length is about 1mm. Other lengths are contemplated.

In some embodiments, the lumens 264, 261 of the main body and distal segments 250, 252 receive a portion of the lighting system 204 in the manner described below. In this embodiment, the cannula 202 is sized to penetrate and be used within an eye globe and may be used to scan tissue of a patient, including eye tissue of a patient, such as retina tissue.

The lighting system 204 comprises the light source 108, which in the embodiment shown, is carried on the console 102 (Fig. 1), a lens 210, and a fiber 214.

The lens 210 is carried within the distal segment 252 of the cannula 202 and is shown in greater detail in Figs. 3 and 4. Fig. 3 shows the distal segment 252 of the cannula 202 and the distal portion 256 of the main body segment 250 of the cannula 202. Fig. 4 shows a side view of the lens 210 independent of the cannula 202.

The lens 210 includes a proximal side 220, a distal side 222, and an outer periphery 224. The proximal side 220 is disposed in the cannula 202, faces the fiber 214, and is typically disposed only slyghtly spaced from the fiber 214. The distal side 222 is disposed outside the cannula 202 or facing the direction of the opening 262 of the cannula 202 and faces tissue to be imaged when in use. The outer periphery 224 extends between the proximal side 220 and the distal side 222. In some embodiments, the outer periphery 224 is cylindrically shaped, while in other embodiments, it is rectangular, oval or otherwise shaped. The outer periphery 224 may be arranged to interface with an inner surface of the cannula 202. The lens 210 also includes an optical axis 226, as shown in Fig. 4. In Fig. 3, the optical axis 226 is coaxial with the central axis 270 of the distal segment 252.

Referring to Fig. 4, the proximal and distal sides 220, 222 of the lens 210 may cooperate to pass light from the light source out of the fiber and to pass light reflected back from the tissue. In some embodiments, the proximal and distal sides 220, 222 are planar surfaces forming substantially parallel planes. The lens 210 may be any size suitable for use in the OCT probe 106, and in some embodiments, has a length less than about 2mm, and in some embodiments, has a length of about 1mm, for example. The width or diameter of the lens 210 may be, for example, between 0.2mm and 2mm. In some smaller gauge probes, the lens 210 has a width or diameter below about 0.6mm or less, and in some probes, about 0.3mm or less. In some embodiments, the lens 210 is a gradient index (GRIN) lens having surfaces through which light from the fiber 214 may pass. Depending upon the embodiment, the gradient index may be spherical, axial, or radial. In another embodiment, the lens 210 is a spherical lens. Other lens shapes may be used.

In the example shown, since there is no longer a need to undergo the difficult process of grinding or polishing an angle onto the lens 210, the proximal and distal sides 220, 222 of the lens 210 form a substantially right angle relative to the outer periphery 224. As used herein, a substantially right angle is intended to include angles resulting from manufacturing tolerances, and may include angles from about 88 to 92 degrees.

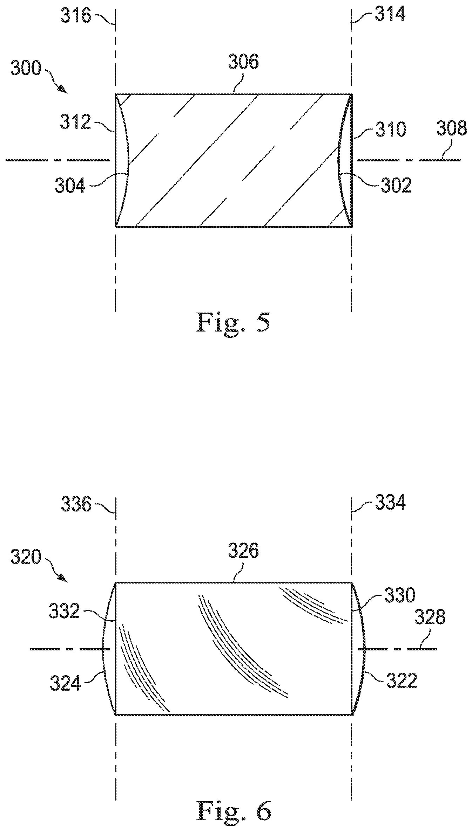

Fig. 5 shows an alternative lens 300 having a distal side 302 and a proximal side 304 formed as convex surfaces. An outer periphery 306 extends between the distal and proximal sides 302, 304 in the manner discussed with reference to the lens 210, and an optical axis 308 extends therethrough substantially parallel to the outer periphery 306 of the lens 300. Fig. 6 shows an alternative lens 320 having a distal side 322 and a proximal side 324 formed as concave surfaces, and having an outer periphery 326 extending between the distal and proximal sides 322, 324. An optical axis 328 extends therethrough substantially parallel to the outer periphery 326 of the lens 320. In order to show the convex distal and proximal sides 302, 304 and peripheral edge 306, the lens 300 in Fig. 5 is shown in cross-section. The lens 320 in Fig. 6 is shown as a side view since the distal and proximal sides 322, 324 can be seen.

These lenses 300, 320 may be disposed in place of the lens 210 in the exemplary probes described herein, including the probe 106 in Fig. 2. In each instance of these examples, however, the lenses 300, 320 respectively include a distal peripheral edge 310, 330 where the outer peripheries 306, 326 intersect the respective distal sides 302, 322. Likewise, the lenses 300, 320 respectively include a proximal peripheral edge 312, 332 where the outer peripheries 306, 326 intersect the respective proximal sides 304, 324. The lens 210, with its substantially planar sides, also includes distal and proximal peripheral edges.

Referring first to Fig. 5, the distal peripheral edge 310 lies along a plane 314 that is substantially normal to the optical axis 308. Likewise, the proximal peripheral edge 312 lies along a plane 316 that is substantially normal to the optical axis 308. Accordingly, the planes 314 and 316 are substantially parallel. In addition, the planes 314, 316 are disposed at substantially right angles relative to the outer periphery 306 of the lens 300. Having parallel distal and proximal sides may simplify manufacturing and reduce costs of the lens.

Likewise, the distal peripheral edge 330 of the lens 320 in Fig. 6 lies along a plane 334 that is substantially normal to the optical axis 328, and the proximal peripheral edge 332 lies along a plane 336 that is substantially normal to the optical axis 328. Accordingly, the planes 334 and 336 are substantially parallel. In addition, the planes 334, 336 are disposed at substantially right angles relative to the outer periphery 326 of the lens 320.

Yet other contemplated embodiments include a combination of lens shapes, such as a lens having a convex end and a concave end, having a concave end and a planar end, or having a convex end and a planar end, for example.

Returning now to Fig. 2, the fiber 214 is configured to transmit light from the light source 108 to the lens 210, and ultimately to the tissue under observation. The fiber 214 in this embodiment is a single fiber, while in other embodiments, the fiber 214 is a fiber bundle. A proximal end (not shown) of the fiber 214 is disposed adjacent the light source 108, while a distal end 218 is disposed adjacent the lens 210 in a manner directing light through the lens 210. In the embodiment shown, the fiber 214 is not directly connected to the lens 210, and the lens 210 is fixed in place relative to the cannula 202. Accordingly, the fiber 214 may move relative to the cannula 202, and the lens 210. The distal end 218 of the fiber 214 is positioned a pre-determined distance from a face of the lens 210 to achieve prescribed optical performance.

In the embodiment shown, the actuation system 206 is disposed primarily within the probe housing 200. The actuation system 206 moves the fiber 214 of the lighting system 204 relative to the cannula 202 in order to provide either one or two dimensional directional scanning to create 2D or 3D images with the OCT imaging system 100. The actuation system 206 may include a microelectrical mechanical systems (MEMS) micromoter, a linear motor, a piezoelectric motor, an electro-magnetic motor, a pneumatic piston, diaphragms, electrical solenoid, or other such element. The actuation system 206 is configured to impart a force on the fiber 214 to physically displace an end of the fiber 214.

The actuation system 206 is configured to pivot the fiber 214 in a manner that causes the end of the fiber 214 to displace relative to the cannula 202, and thereby move the fiber 214 in at least a single plane to perform a scan. Scanning allows light to be taken over an area of the tissue being evaluated, rather than a specific spot or point on the tissue. The scan is then converted into a 2D or 3D image by the OCT system 100 (Fig. 1) that may be evaluated by the health care provider.

In use, it may be desirable to maintain a continuous distance between the fiber 214 and the lens 210, even as the fiber 214 pivots or displaces relative to the lens 210. As can be seen in Fig. 3, moving the fiber in a plane that includes both the main body segment axis 203 and the distal segment axis 170 would result in changes in distance between the fiber 214 and the lens 210, due to the angled orientation of the lens 210. To address this, some embodiments are configured so that the actuation system 206 pivots or displaces the fiber 214 in the cannula 202 in a direction that is not aligned with a plane that includes both the main body segment axis 203 and the distal segment axis 170. In some embodiments, the actuation system 206 pivots the fiber in a direction directly transverse to a plane that includes both the main body segment axis 203 and the distal segment axis 170. In so doing, the angled orientation of the lens surface will have little or no impact on the distance between the pivoting fiber 214 and the distal lens surface 220. That is, even during pivoting, the fiber tip 218 and the lens 210 will be maintained apart at substantially the same distance. As such, an accurate scan of patient tissue can be captured.

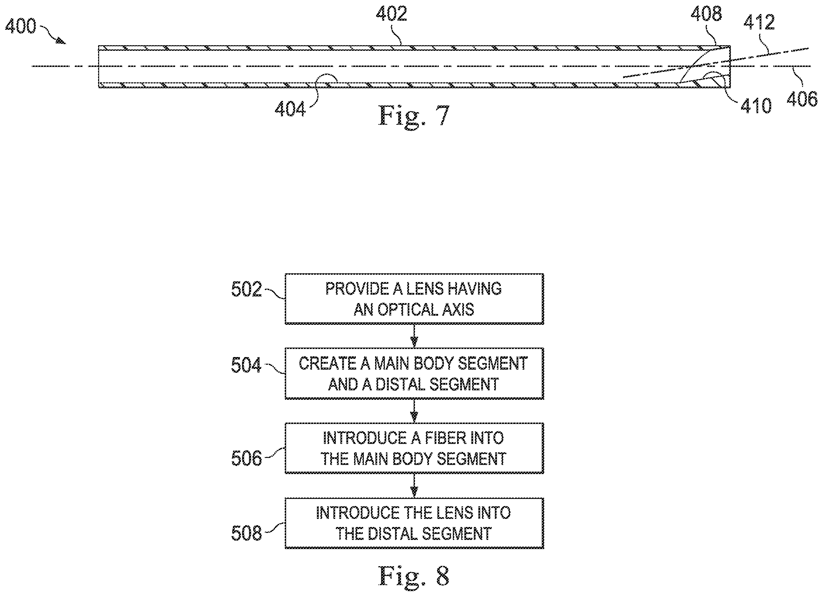

Fig. 7 discloses an alternative embodiment of a cannula, referenced here by the numeral 400. The cannula 400 may be used in place of the cannula 202. Here, the cannula 400 includes a main body segment 402 having a lumen 404 that defines a main body axis 406 and includes a distal segment 408 having a lumen 410 that defines a distal segment axis 412. The lumens 404, 410 are angled in the manner discussed above, and as such, the axes 406, 412 are not coaxial. Any of the lenses described herein may be used in the cannula 400. In this embodiment, the cannula 400 includes a cylindrical outer surface extending along both main body segment 402 and the distal segment 408.

The flow chart of Fig. 8 shows an exemplary method of manufacturing the probe 106 to have the angled lumen and the easier to assembly lens. In this example, the method starts at a step 502.

At a step 502, a lens usable in an OCT probe and including an optical axis is provided. Depending on the embodiment, the lens is configured to have a distal surface and a proximal surface that are substantially parallel to one another. In some embodiments, the lens includes a peripheral surface that extends in a direction substantially perpendicular to the distal and proximal surfaces. When the lens is concave or convex or otherwise in a nonplanar configuration, the peripheral surface may be perpendicular to a plane that includes the peripheral edges of the distal and proximal surfaces.

At a step 504, the manufacturer creates the main body segment and the distal segment of the cannula. In some embodiments, this includes forming a bend in the cannula that separates the main body segment and the distal segment. This may include bending the distal end of the cannula to form the distal segment. In some embodiments, the distal segment is bent to form an angle within a range of about 4 to 20 degrees, 7 to 9 degrees, or about 8 degrees. In other embodiments, the angled distal segment is formed through machining, extrusion, or other process.

At a step 506, the fiber is introduced into the main body portion of the cannula. This may be done by introducing the optical fiber through the proximal end of the main body portion of the cannula.

At a step 508, the lens is introduced into the distal segment of the cannula 202. The lens may be inserted through the distal opening of the cannula until it abuts the main body segment. Once introduced, the lens may be secured within the distal segment. This may be accomplished using an adhesive, an interference fit, or other securing method. The lens may be oriented to be spaced a specific distance from a distal end of the fiber so that the fiber may be laterally displaced relative to the lens to create an OCT scan.

In operation, a health care provider controls the OCT probe 106 at the console 102 and then orients the OCT probe 106 at a location adjacent tissue to be evaluated in a manner known in the art. With the OCT probe 106 at its desired location, the OCT probe 106 is activated to begin a scanning procedure. To do this, the actuation system 206 operates to physically displace the fiber 214 relative to the main body portion of the cannula 202 and to the lens in a back and forth motion. In some embodiments, the fiber moves in a direction lateral to a plane that passes through the axes of the main body segment and the distal segment.

Because of the angled cannula, the lens may be manufactured in an easier and potentially less expensive manner by reducing the necessity of forming the proximal surface of the lens at an angle relative to the distal surface. In some instances, this proximal surface of the lens is formed at a right angle relative to the outer periphery of the lens. Because the lens is maintained at an angle by the cannula, the lens may be manufactured using less expensive processes, and still provides the advantages obtained by an angled lens because light reflected by the proximal surface of the lens is angled away from the fiber. This allows the OCT image to be generated with less noise due to light feedback, but also may reduce costs of the probe, resulting in a product that may be less expensive, increasing availability and demand.

10057] Persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An OCT probe (106) for imaging patient tissue, comprising:
a cannula (202) extending from a probe housing (200) and arranged to penetrate patient tissue, the cannula having an elbow formed therein dividing the cannula into a main body segment (250) and a distal segment (252), the main body segment having a lumen defining a first central axis (270), the distal segment having a lumen defining a second central axis that is angled from the first central axis (203);
a selectively displaceable light-carrying fiber (214) disposed within the main body segment of the cannula, the fiber having a distal end and being arranged to emit light from the distal end; and
a lens (210) disposed in the distal segment of the cannula, the lens comprising a proximal side and a distal side, the proximal side being disposed relative to the fiber so that light emitted through the fiber and reflected from the proximal side reflects at an angle misaligned with the fiber,
wherein the OCT probe further comprises an actuation system (206) configured to displace the fiber in the lumen of the main body segment in a direction transverse to a plane through the first central axis and the second central axis.

2. The OCT probe of claim 1, wherein the cannula has an outer diameter in the range of about 1-3mm.

3. The OCT probe of claim 1, wherein the first central axis and the second central axis form an angle between about 7 degrees and 9 degrees.

4. The OCT probe of claim 1, wherein the length of the distal segment measured along the second central axis is within a range of about 0.5 and 3mm.

5. The OCT probe of claim 1, wherein the distal side of the lens includes a perimeter edge in a first plane and the proximal side of the lens includes a perimeter edge in a second plane, the first and second planes being substantially parallel to each other.

6. The OCT probe of claim 5, wherein the lens comprises an outer periphery, the proximal side and the distal side being arranged so that the first and second planes form substantially a right angle with the outer periphery.

7. The OCT probe of claim 1, wherein at least one of the proximal side and the distal side is planar.

8. The OCT probe of claim 1, wherein the cannula is sized to penetrate the globe of an eye to image tissue in the eye.

9. The OCT probe of claim 1, wherein the lens has a width less than about 2mm and a length less than about 2mm.

10. The OCT probe of claim 1, wherein the elbow is a bend in the cannula.

## Patentansprüche

1. OCT-Sonde (106) zum Abbilden von Patientengewebe, umfassend:
eine Kanüle (202), die sich von einem Sondengehäuse (200) erstreckt und dazu angeordnet ist, ein Patientengewebe zu durchdringen, wobei die Kanüle einen Ellbogen darin gebildet aufweist, der die Kanüle in ein Hauptkörpersegment (250) und ein distales Segment (252) unterteilt, wobei das Hauptkörpersegment ein Lumen aufweist, das eine erste Mittelachse (270) definiert, wobei das distale Segment ein Lumen aufweist, das eine zweite Mittelachse aufweist, die von der ersten Mittelachse (203) abgewinkelt ist;
eine selektiv verlagerbare lichtübertragende Faser (214), die innerhalb des Hauptkörpersegments der Kanüle angeordnet ist, wobei die Faser ein distales Ende aufweist und dazu angeordnet ist, Licht von dem distalen Ende abzugeben; und
eine Linse (210), die in dem distalen Segment der Kanüle angeordnet ist, wobei die Linse eine proximale Seite und eine distale Seite umfasst, wobei die proximale Seite bezogen auf die Faser derart angeordnet ist, dass Licht, das durch die Faser abgegeben und von der proximalen Seite reflektiert wurde, in einem zur Faser versetzten Winkel reflektiert wird,
wobei die OCT-Sonde ferner ein Betätigungssystem (206) umfasst, das dazu ausgelegt ist, die Faser in dem Lumen des Hauptkörpersegments in eine Richtung schräg zu einer Ebene durch die erste Mittelachse und die zweite Mittelachse zu verlagern.

2. OCT-Sonde nach Anspruch 1, wobei die Kanüle einen Außendurchmesser im Bereich von ungefähr 1-3 mm aufweist.

3. OCT-Sonde nach Anspruch 1, wobei die erste Mittelachse und die zweite Mittelachse einen Winkel zwischen ungefähr 7 Grad und 9 Grad bilden.

4. OCT-Sonde nach Anspruch 1, wobei die Länge des distalen Segments, die entlang der zweiten Mittelachse gemessen wurde, in einem Bereich von ungefähr 0,5 und 3 mm liegt.

5. OCT-Sonde nach Anspruch 1, wobei die distale Seite der Linse einen Umfangsrand in einer ersten Ebene umfasst und die proximale Seite der Linse einen Umfangsrand in einer zweiten Ebene umfasst, wobei die erste und die zweite Ebene im Wesentlichen parallel zueinander verlaufen.

6. OCT-Sonde nach Anspruch 5, wobei die Linse einen Außenumfang umfasst, wobei die proximale Seite und die distale Seite derart angeordnet sind, dass die erste und die zweite Ebene im Wesentlichen einen rechten Winkel mit dem Außenumfang bilden.

7. OCT-Sonde nach Anspruch 1, wobei mindestens eine der proximalen Seite und der distalen Seite eben ist.

8. OCT-Sonde nach Anspruch 1, wobei die Kanüle in der Größe so festgelegt ist, dass sie einen Augapfel durchdringen kann, um das Gewebe in dem Auge abzubilden.

9. OCT-Sonde nach Anspruch 1, wobei die Linse eine Breite aufweist, die kleiner ist als ungefähr 2 mm, und eine Länge, die kleiner ist als ungefähr 2 mm.

10. OCT-Sonde nach Anspruch 1, wobei der Ellbogen eine Krümmung in der Kanüle ist.

## Revendications

1. Sonde OCT (106) destinée à imager un tissu d'un patient, comprenant :
une canule (202) s'étendant depuis un boîtier de sonde (200) et agencée pour pénétrer dans un tissu du patient, la canule ayant un coude formé à l'intérieur qui divise la canule en un segment de corps principal (250) et un segment distal (252), le segment de corps principal ayant une lumière définissant un premier axe central (270), le segment distal ayant une lumière définissant un deuxième axe central qui est incliné par rapport au premier axe central (203) ;
une fibre transportant la lumière sélectivement déplaçable (214) disposée à l'intérieur du segment de corps principal de la canule, la fibre ayant une extrémité distale et étant agencée pour émettre de la lumière depuis l'extrémité distale ; et
une lentille (210) disposée dans le segment distal de la canule, la lentille comprenant un côté proximal et un côté distal, le côté proximal étant disposé par rapport à la fibre de telle sorte que la lumière émise à travers la fibre et réfléchie depuis le côté maximal se réfléchit à un angle non aligné avec la fibre,
la sonde OCT comprenant en outre un système d'actionnement (206) configuré pour déplacer la fibre dans la lumière du segment de corps principal dans une direction transversale à un plan passant par le premier axe central et le deuxième axe central.

2. Sonde OCT de la revendication 1, dans laquelle la canule a un diamètre extérieur dans la gamme d'environ 1-3 mm.

3. Sonde OCT de la revendication 1, dans laquelle le premier axe central et le deuxième axe central forment un angle entre environ 7 degrés et 9 degrés.

4. Sonde OCT de la revendication 1, dans laquelle la longueur du segment distal mesurée le long du deuxième axe central se situe dans une gamme d'environ 0,5 à 3 mm.

5. Sonde OCT de la revendication 1, dans laquelle le côté distal de la lentille comporte un bord périmétrique dans un premier plan et le côté proximal de la lentille comporte un bord périmétrique dans un deuxième plan, les premier et deuxième plans étant sensiblement parallèles l'un à l'autre.

6. Sonde OCT de la revendication 5, dans laquelle la lentille comprend une périphérie externe, le côté proximal et le côté distal étant agencés de telle sorte que les premier et deuxième plans forment un angle sensiblement droit avec la périphérie externe.

7. Sonde OCT de la revendication 1, dans laquelle le côté proximal et/ou le côté distal sont plans.

8. Sonde OCT de la revendication 1, dans laquelle la canule est dimensionnée pour pénétrer dans le globe d'un oeil pour imager un tissu dans l'oeil.

9. Sonde OCT de la revendication 1, dans laquelle la lentille a une largeur inférieure à environ 2 mm et une longueur inférieure à environ 2 mm.

10. Sonde OCT de la revendication 1, dans laquelle le coude est un pli dans la canule.
